# EUROPÄISCHE PATENTSCHRIFT

(11) **EP 2 507 388 B1**
(45) Veröffentlichungstag und Bekanntmachung des Hinweises auf die Patenterteilung: **12.10.2016**
(21) Anmeldenummer: 10794912.5
(22) Anmeldetag: 23.11.2010
(51) Int. Cl.: C12Q 1/68

(54) **SELEKTIVE ANREICHERUNG NICHT -METHYLIERTER NUKLEINSÄUREN**
SELECTIVE ENRICHMENT OF NON-METHYLATED NUCLEIC ACIDS
ENRICHISSEMENT SÉLECTIF D'ACIDES NUCLÉIQUES NON MÉTHYLÉS

(30) Priorität: 04.12.2009 DE 102009057702
(43) Veröffentlichungstag der Anmeldung: 10.10.2012
(73) Patentinhaber: Qiagen GmbH, 40724 Hilden (DE)
(72) Erfinder: KORFHAGE, Christian, 40764 Langenfeld (DE); MEIER, Andreas, 40227 Düsseldorf (DE)
(74) Vertreter: CH Kilger Anwaltspartnerschaft mbB
(86) Internationale Anmeldenummer: PCT/EP2010/068006
(87) Internationale Veröffentlichungsnummer: WO 2011/067133

(56) Entgegenhaltungen:
- WO-A1-2009/002891
- WO-A1-2009/098298
- WO-A2-02/081749
- US-A1- 2005 202 490
- US-A1- 2005 260 630
- US-A1- 2005 272 065
- US-A1- 2006 204 988
- GONZALGO M L ET AL: "Identification and characterization of differentially methylated regions of genomic DNA by methylation-sensitive arbitrarily primed PCR", CANCER RESEARCH, AMERICAN ASSOCIATION FOR CANCER REREARCH, US, Bd. 57, Nr. 4, 15. Februar 1997 (1997-02-15), Seiten 594-599, XP008059557, ISSN: 0008-5472
- KOHNO TAKASHI ET AL: "identification of CpG islands hypermethylated in human lung cancer the arbitrarily primed-PCR method", HUMAN GENETICS, SPRINGER, BERLIN, DE, Bd. 102, Nr. 3, 1. März 1998 (1998-03-01), Seiten 258-264, XP002211910, ISSN: 0340-6717, DOI: DOI:10.1007/S004390050689
- HUANG T H ET AL: "Identification of DNA methylation markers for human breast carcinomas using the methylation-sensitive restriction fingerprinting technique.", CANCER RESEARCH 15 MAR 1997 LNKD- PUBMED:9067264, Bd. 57, Nr. 6, 15. März 1997 (1997-03-15), Seiten 1030-1034, XP002644914, ISSN: 0008-5472
- KANEDA A ET AL: "METHYLATION-SENSITIVE REPRESENTATIONAL DIFFERENCE ANALYSIS AND ITS APPLICATION TO CANCER RESEARCH", ANNALS OF THE NEW YORK ACADEMY OF SCIENCES, NEW YORK ACADEMY OF SCIENCES, NEW YORK, NY, US, Bd. 983, 1. März 2003 (2003-03-01), Seiten 131-141, XP009067900, ISSN: 0077-8923, DOI: DOI:10.1111/J.1749-6632.2003.TB05968.X
- HUANG TIM HUI-MING ET AL: "Methylation profiling of CpG islands in human breast cancer cells", HUMAN MOLECULAR GENETICS, OXFORD UNIVERSITY PRESS, SURREY, Bd. 8, Nr. 3, 1. März 1999 (1999-03-01), Seiten 459-470, XP002175857, ISSN: 0964-6906, DOI: DOI:10.1093/HMG/8.3.459
- WALKER G T ET AL: "Isothermal in vitro amplification of DNA by a restriction enzyme/DNA polymerase system", PROCEEDINGS OF THE NATIONAL ACADEMY OF SCIENCES, NATIONAL ACADEMY OF SCIENCES, US, vol. 89, 1 January 1992 (1992-01-01), pages 392-396, XP002905182, ISSN: 0027-8424, DOI: 10.1073/PNAS.89.1.392
- Anonymous: "Digestion of PCR Products", http://www.fermentas.com/techinfo /RE/restrdigpcr.htm, 20 September 2002 (2002-09-20), XP055167191, Retrieved from the Internet: URL:http://www.protocol-online.org/cgi-bin /prot/view_cache.cgi?ID=749 [retrieved on 2015-02-04]
- QIAGEN: 'Whole Genome Amplification - REPLI-g Principle and Procedures - QIAGEN', [Online] 01 Januar 2015, XP055202952 Gefunden im Internet: <URL:https://www.qiagen.com/de/resources/te chnologies/wga/replig-principal-procedure/? Print=1 ttab=isProductVisible> [gefunden am 2015-07-16]
- ANONYMOUS: 'Product info: Glu I', [Online] 16 Mai 2015, NOVOSIBIRSK, RUSSIA, XP055202990 Gefunden im Internet: <URL:http://www.sibenzyme.com/info642.php> [gefunden am 2015-07-16]

### SEQUENCE LISTING

<110> Qiagen GmbH
<120> SELEKTIVE ANREICHERUNG NICHT-METHYLIERTER NUKLEINSÄUREN
<130> R2672 PCT BLN
<150> DE 10 2009 057 702.5
   <151> 2009-12-04
<160> 14
<170> PatentIn version 3.5
<210> 1
   <211> 20
   <212> DNA
   <213> Artificial Sequence
<220>
   <223> Primer, Locus a
<400> 1
   ttcccactca aaactcccac 20
<210> 2
   <211> 20
   <212> DNA
   <213> Artificial Sequence
<220>
   <223> Primer, Locus a
<400> 2
   acaggaatga gggcagctaa 20
<210> 3
   <211> 21
   <212> DNA
   <213> Artificial Sequence
<220>
   <223> Primer, Locus b
<400> 3
   tgcccgcgtc cgtccgtgaa a 21
<210> 4
   <211> 22
   <212> DNA
   <213> Artificial Sequence
<220>
   <223> Primer, Locus b
<400> 4
   agtctccgtc gccgtcctcg tc 22
<210> 5
   <211> 23
   <212> DNA
   <213> Artificial Sequence
<220>
   <223> Primer, Locus c
<400> 5
   ggtaggatga ttctagaatg aca 23
<210> 6
   <211> 20
   <212> DNA
   <213> Artificial Sequence
<220>
   <223> Primer, Locus c
<400> 6
   gcccaaattg gcttcttttt 20
<210> 7
   <211> 22
   <212> DNA
   <213> Artificial Sequence
<220>
   <223> Primer, Locus d
<400> 7
   gtctttagct gctgaggaaa tg 22
<210> 8
   <211> 21
   <212> DNA
   <213> Artificial Sequence
<220>
   <223> Primer, Locus d
<400> 8
   agcagaattc tgcacatgac g 21
<210> 9
   <211> 19
   <212> DNA
   <213> Artificial Sequence
<220>
   <223> Primer, Locus e
<400> 9
   caactggccc tgtcgttcc 19
<210> 10
   <211> 20
   <212> DNA
   <213> Artificial Sequence
<220>
   <223> Primer, Locus e
<400> 10
   ccatgttgct gacccggtag 20
<210> 11
   <211> 22
   <212> DNA
   <213> Artificial Sequence
<220>
   <223> Primer, Locus f
<400> 11
   actggttgga gttgtggaga cg 22
<210> 12
   <211> 21
   <212> DNA
   <213> Artificial Sequence
<220>
   <223> Primer, Locus f
<400> 12
   tggaatgctt gaaggctgct c 21
<210> 13
   <211> 21
   <212> DNA
   <213> Artificial Sequence
<220>
   <223> Primer, Locus g
<400> 13
   aactgaatgg cagtgaaaac a 21
<210> 14
   <211> 19
   <212> DNA
   <213> Artificial Sequence
<220>
   <223> Primer, Locus g
<400> 14
   ccctagcctg tcattgctg 19

## Beschreibung

### Technisches Gebiet der Erfindung

Die vorliegende Erfindung betrifft das Gebiet der Biologie und Chemie, insbesondere der Molekularbiologie. Im Speziellen betrifft die Erfindung die Amplifikation von nicht-methylierte Bereichen von Nukleinsäuren sowie die Analyse von Methylierungsmustern in Nukleinsäuren.

### Hintergrund der Erfindung

Methylierung ist eine häufig vorkommende chemische Veränderung der DNA, bei der Methylgruppen auf Nucleobasen übertragen wurden, z.B. an der Kohlenstoff-5-Position des Pyrimidinrings des Cytosins. Dies geschieht im Regelfall durch spezielle DNA-Methyltransferasen, entweder *de novo* oder zur Aufrechterhaltung eines bestehenden Methylierungsmusters etwa bei der Replikation von DNA.

DNA-Methylierung kann mehrere Funktionen haben: Zum Beispiel kann sie Prokaryoten dazu dienen, eigene DNA von fremder, eingeschleuster DNA zu unterscheiden. Zudem spielt sie unter anderem eine wichtige Rolle bei der Fehlerkorrektur während der DNA-Synthese in Prokaryoten, um zwischen dem ursprünglichen (Templat-)Strang und dem neu synthetisierten Strang zu unterscheiden. Viele Prokaryoten haben DNA-Methyltransferasen, die eigene DNA an oder in der Nähe von bestimmten Signalsequenzen methylieren. Fremde, unmethylierte DNA kann in diesen Organismen von speziellen methylierungs-sensitiven Restriktionsendonukleasen an oder in der nähe der Signalsequenzen geschnitten und somit abgebaut werden.

Neben den erwähnten methylierungs-sensitiven Endonukleasen, die nur nicht-methylierte Bereiche schneiden, gibt es auch methylierungsabhängige Endonukleasen, die nur an oder neben bestimmten methylierten Sequenzen schneiden.

In Eukaryoten bietet die Methylierung der DNA eine zusätzliche Informationsebene, so zum Beispiel um aktive von inaktiven Bereichen des Genoms zu unterscheiden. Insbesondere bei der differentiellen Genexpression spielen Methylierungsmuster eine besondere Rolle und sind daher auch relevant in der Entstehung von Tumoren.

Zur Aufklärung von Methylierungsmustern in DNA sind dem Fachmann eine Reihe von Verfahren aus dem Stand der Technik bekannt: In der Bisulfit-Sequenzierung zum Beispiel wird zunächst die zu analysierende DNA mit Bisulfit umgesetzt, so dass die nichtmethylierten Cysteine in Uracil umgewandelt werden, gefolgt von einer Amplifikation mittels PCR sowie DNA-Sequenzierung. Aus den Sequenzunterschieden der Bisulfit-behandelten und nicht-Bisulfit-behandelten DNA kann auf das zugrundeliegende Methylierungsmuster geschlossen werden. Alternativ kann auch eine methylierungs-spezifische PCR (*Methylation Specific PCR*; MSP) zur Analyse der Bisulfit-behandelten DNA eingesetzt werden, wobei methylierungspezifische Primer eingesetzt werden, d.h. Primer, die komplementär zur unkonvertierten Sequenz sind. Alternativ zur Bisulfit-Methode können andere Verfahren, wie die methylierungsspezifische Restriktionsanalyse oder methylierte-DNA-Immunopräzipitation (*methylated DNA immunoprecipitation*; MeDIP) eingesetzt werden.

Diese Verfahren sind auf die Analyse der Methylierung von definierten Sequenzbereichen sowie der Quantifizierung des Methylierungsgrades von definierten Sequenzbereichen ausgerichtet.

Das Dokument US2005/0272065 A1 offenbart Verfahren zur Bestimmung des Methylierungsstatus an einem Locus, umfassend den Verdau mit einer methylierungsabhängigen Nuklease und die Amplifizierung unmethylierter DNA mit Zufallsprimern.

### Beschreibung der Erfindung

Die vorliegende Erfindung betrifft ein Verfahren mit dem sich Aussagen über das globale Methylierungsmuster einer DNA treffen lassen. Das erfindungsgemäße Verfahren ist also primär auf die Identifikation von Genomabschnitten, die methylierte Bereiche enthalten, ausgerichtet. In besonders bevorzugten Ausführungsformen ist die Erfindung auf die Identifikation von Genomabschnitten, die methylierte Bereiche enthalten, ausgerichtet und nicht auf die Feststellung von Methylierungszuständen bestimmter einzelner Basen.

Mit dem erfindungsgemäßen Verfahren kann auch eine selektive Präparation von nichtmethylierter DNA vorgenommen werden.

Das Verfahren besteht aus mehreren Teilschritten:
(1) DNA, vorzugsweise genomische DNA, wird mit einer methylierungsabhängigen Nukleasen verdaut.
(2) Die DNA wird nach dem Verdau mittels eines Amplifikationsverfahrens, vorzugsweise eines *Random-primed* Sequenz-Amplifikationsverfahrens vervielfältigt, so dass nur die nicht-methylierten DNA Abschnitte - also solche, die nicht geschnitten wurden - vervielfältigt werden können. Ergebnis der Amplifikation ist die vervielfältigte DNA ohne solche Abschnitte, die vorher durch die methylierungsabhängige Nuldease geschnitten wurden. Dementsprechend führt das Verfahren zu einer selektiven Anreicherung und Vervielfältigung von Sequenzschnitten, die nicht methyliert sind.
(3) Optional kann im Anschluss eine quantitative Analyse der Kopiezahl von Sequenzabschnitten in der verfahrensgemäß selektierten und amplifizierten DNA durchgeführt werden.

Letzteres kann der Analyse des Methylierungsmusters einer DNA bzw. Teilabschnitten davon dienen, d.h. zu welchem Grad eine DNA oder ein definierter Teil davon ursprünglich methyliert war.

Das Verfahren der vorliegenden Erfindung ist somit komplementär zu anderen Verfahren zur Analyse der Methylierung von Nukleinsäuren.

Das erfindungsgemäße Verfahren kann bei der Identifizierung von Genomabschnitte helfen, die methyliert sind. Das Verfahren erlaubt durch die Amplifikation der selektierten Sequenzen eine globale Methylierungsanalyse ohne die genaue Stelle der Methylierung kennen zu müssen.

Somit betrifft die Erfindung ein Verfahren zur selektiven Amplifikation von nichtmethylierten Sequenzen einer DNA umfassend die Schritte
(i) Bereitstellen einer Probe umfassend eine DNA, die an mindestens einer Stelle methyliert ist,
(ii) Behandlung der DNA in der Probe mit einer methylierungsabhängigen Nuklease, und
(iii) Isothermale Random Primed Sequenz-Amplifikation der mit der methylierungsabhängigen Nuklease geschnittenen DNA, wobei die

Schritte (ii) und (iii) gleichzeitig (simultan) durchgeführt werden.

Eine Nuklease ist ein Enzym, das hydrolytisch eine Nukleinsäure (z.B. genomische DNA) teilt. Dabei werden die Phosphodiesterbindungen hydrolytisch gespalten. Bevorzugt sind im Zusammenhang der Erfindung Endonukleasen. Eine Nuklease ist dann methylierungsabhängig, wenn das Enzym nur an methylierten Stellen binden kann oder nur methylierte Stellen spalten kann. Zu solchen methylierungsabhängigen Nukleasen gehören z.B. die Enzyme McrBC, McrA und MrrA. McrA schneidet m5CG-methylierte DNA, McrBC schneidet (A/G)m5C-methylierte DNA und MrrA schneidet m6N Adenin-methylierte DNA. Die methylierungsabhängige Nuklease ist vorzugsweise ausgewählt aus der Gruppe bestehend aus McrBC, McrA, DpnI, BisI, BlsI, GlaI, GluI, MalI und PcsI. Solche Nukleasen sind z.B. in Chmuzh et al. (2005) (BMC Microbiology 6:40i), Tarasova et al. (2008) (BMC Molecular Biology 9:7), Chernukhin et al. (2007a) (Ovchinnikov bulletin of biotechnology and physical and chemical biology V.3, No 1, pp 28-33) und Chernukhin et al. (2007b) (Ovchinnikov bulletin of biotechnology and physical and chemical biology V.3, No 2, pp 13-17) beschrieben. Bevorzugte methylierungsabhängige Nukleasen im Zusammenhang der vorliegenden Erfindung sind McrBC und McrA, besonders bevorzugt ist McrBC. McrBC ist kommerziell erhältlich, z.B. über New England Biolabs Inc., Ipswich, MA, USA. Darüber hinaus können auch Homologe der genannten Nukleasen verwendet werden, z.B. die in Fukuda (2008) (Genome Biol. 9(11):R163) beschriebenen McrBC-homologen Enzymsysteme. Auch LlaJI wurde als McrBC-homolog beschrieben (O'Driscoll (2006), BMC Microbiology 2006, 6:40i). Weiterhin geeignet sind in besonderen Ausführungsformen der vorliegenden Erfindung Nukleasen, deren Spezifität z.B. durch Verwendung neuer Pufferbedingungen, Modifikation(en), Aminosäureaustausch(en) oder andere Manipulationen so verändert wurde, dass sie hemimethylierte oder komplett methylierte Bereiche schneiden können. Solche Enzyme sind z.B. in Formenkov et al (2008) (Anal. Biochemistry 381: 135-141) beschrieben.

Alternativ kann auch einer methylierten Base durch eine DNA Glycosylase aus der DNA herausgeschnitten werden. Dies führt dazu, dass bei einer anschließende Amplifikationsreaktion diese Stelle nicht amplifiziert werden kann. So kann zum Beispiel das 5-methyl-Cytosin durch eine 5-methyl-Cytosin DNA Glycosylase ausgeschnitten werden. Die Effizienz des Amplifikationsstopps an der abasischen Stelle kann durch eine entsprechende Lyase unterstützt werden, die an der abasischen Stelle das Zucker-Phosphat Rückgrat der DNA schneidet. Das Verfahren kann auch hier zu einem Strangbruch führen, z.B. durch die Verwendung von Enzymen (z.B. Lyasen) oder entsprechender Reaktionsbedingungen.

Alle genannten Enzyme und Enzymsysteme können somit unter geeigneten Bedingungen als methylierungsabhängige Nukleasen im weiteren Sinne betrachtet werden, sofern sie die entsprechende Aktivität aufweisen.

Die Amplifikation kann dabei grundsätzlich mittels isothermalen oder nicht-isothermalen Verfahren durchgeführt werden. Bekannte isothermale Amplifikationsverfahren sind z.B. *Strand Displacement-*Amplifikation (SDA), *Multiple Displacement*-Amplifikation (MDA), *Rolling-Circle*-Amplifikation (RCA), *Loop mediated Isothermal Amplification* (LAMP), *Transcription Mediated Amplification* (TMA), *Helicase-dependent Amplification* (HDA), *SMart*-*Amplification Pracess* (SMAP), *Single-Primer-Isothermal-Amplification* (SPIA) . Bekannte nicht-isothermale Amplifikationsverfahren sind z.B. die Ligase-Kettenreaktion (LCR) sowie die Polymerase-Kettenreaktion (PCR). Angewendet werden im Zusammenhang der
vorliegenden Erfindung *Random-Primed* Sequenz-Amplifikationsverfahren. Diese können isothermal oder nicht isothermal sein. Beispiele von nicht-isothermalen Random-Primed Sequenz-Amplifikationsverfahren sind *Random primed*-PCR-Verfahren wie PEP-PCR (primer extension preamplification-PCR), iPEP-PCR (improved primer extension preamplification-PCR), DOP-PCR (Degenerate Oligonucleotid Primer - PCR), Adaptor-Ligation PCR oder Verfahren wie OmniPlex® (Sigma-Aldrich) oder GenomePlex® (Rubicon). Beispiele für isothermale Sequenz-Amplifikationsverfahren der vorliegenden Erfindung sind. die *Strand Displacement-*Reaktionen zu denen z.B. die Strand-Displacement-Amplifikation (SDA) im engeren Sinne gehört sowie die *Multiple Displacement-*Amplifikation (MDA), die *Rolling-Circle-*Amplifikation (RCA), *Single-Primer-Isothermal-Amplification* (SPIA) sowie alle Unterformen dieser Reaktionen, wie *Restriction-aided* RCA (RCA-RCA), MDA mit *nested* Primem, lineare und exponentielle *Strand*-*Displacement*-Reaktionen sowie *Helicase-dependent Amplification* (HDA). Besonders bevorzugte Beispiele von isothermalen *Random-Primed* Sequenz-Amplifikationsverfahren im Rahmen der vorliegenden Erfindung sind MDA und RCA. All diese Verfahren sind dem Fachmann bekannt (vgl. z.B. US 2005/0112639 A1, US 2005/0074804 A1, US 2005/0069939 A1 und US 2005/0069938 A1, sowie Wang G. et al. (2004), Genome Res. Nov;14(11):2357-2366; Milla M.A. et al. (1998), Biotechniques Mar;24(3):392-396; Nagamine K. et al. (2001), Clin Chem. 47(9):1742-1743; Lage J.M. et al. (2003), Genome Res.13(2):294-307 und Vincent M. et al. (2004), EMBO Rep. 5(8):795-800). Unter einer *Strand-Displacement-*Reaktion wird hier jede Reaktion verstanden, bei der eine Polymerase verwendet wird, die eine *Strand*-*Displacement* Aktivität aufweist.

*Strand-Displacement*-Aktivität einer Polymerase bedeutet, dass das eingesetzte Enzym dazu in der Lage ist, einen Nukleinsäure-Doppelstrang in zwei Einzelstränge aufzutrennen. DNA Polymerasen mit *Strand*-*Displacement*-Aktivität, die beispielsweise bei der RCA eingesetzt werden können, sind z.B. Holoenzyme oder Teile von Replikasen aus Viren, Prokaryoten, Eukaryoten, oder Archaeen, Phi 29-artige DNA-Polymerasen, die DNA-Polymerase Klenow exo- und die DNA-Polymerase aus *Bacillus stearothermophilus* mit der Bezeichnung *Bst* exo- "exo-" bedeutet, dass das entsprechende Enzym keine 5'-3'-Exonukleaseaktivität aufweist. Ein bekannter Vertreter der Phi 29-artigen DNA-Polymerasen ist die DNA-Polymerase aus dem Bakteriophagen Phi 29. Andere Phi 29-artige DNA-Polymerasen kommen z.B. in den Phagen Cp-1, PRD-1, Phi 15, Phi 21, PZE, PZA, Nf, M2Y, B103, SF5, GA-1, Cp-5, Cp-7, PR4, PR5, PR722 und L 17 vor. Weitere geeignete DNA-Polymerasen mit *Strand-Displacement-Aktivität* sind dem Fachmann bekannt. Alternativ werden unter DNA-Polymerasen mit *Strand-Displacement-*Aktivität auch solche DNA-Polymerasen ohne *Strand-Displacement-*Aktivität verstanden, wenn neben einer entsprechenden DNA-Polymerase einen Katalysator verwendet wird, beispielsweise ein Protein oder ein Ribozym, welcher die Trennung eines DNA-Doppelstrangs oder die Stabilisierung von DNA-Einzelsträngen ermöglicht. Zu diesen Proteinen gehören z.B. die Helicasen, SSB-Proteine und Rekombinationsproteine, die als Bestandteil größerer Enzymkomplexe wie z.B. Replikasen enthalten sein können. In diesem Falle erzeugt man mit Komponenten zusätzlich zu der Polymerase eine Polymerase mit *Strand-Displacement-*Aktivität*.* Die Polymerasen mit *Strand*-*Displacement* Aktivität können hitzelabil oder hitzestabil sein.

In einer bestimmten Ausführungsform ist die zur Amplifikation verwendete Polymerase mit *Strand-Displacement*-Aktivität eine Phi 29-ähnlichen Polymerase, vorzugsweise eine Polymerase aus einem Phagen ausgewählt aus einer Gruppe von Phagen umfassend Phi 29, Cp-1, PRD-1, Phi 15, Phi 21, PZE, PZA, Nf, M2Y, B103, SF5, GA-1, Cp-5, Cp-7, PR4, PR5, PR722 und L 17. Besonders Bevorzugt ist die Verwendung der Polymerase aus dem Phagen Phi 29.

Es ist für den Fachmann ersichtlich, dass auch die Verwendung von Mischungen von zwei oder mehr Polymerasen mit *Strand-Displacement*-Aktivität möglich ist. Weiter kann auch eine oder mehrere Polymerasen mit *Strand*-*Displacement*-Aktivität mit einer oder mehreren Polymerasen ohne *Strand*-*Displacement*-Aktivität kombiniert werden.

Im Rahmen der vorliegenden Erfindung sind MDA und RCA bevorzugte Amplifikationsverfahren. Bevorzugt ist es weiterhin eine Amplifikation des ganzen Genoms durchzuführen (*Whole Genome Amplification* (WGA)). WGA bedeutet, dass die Templat-DNA grundsätzlich im wesentlichen vollständig amplifiziert werden soll, wobei allerdings erfindungsgemäß nur der nicht-methylierte Teil des Genoms amplifiziert werden soll.
Die Erfindung betrifft in bevorzugten Ausführungsformen somit die Amplifikation von genomischer DNA.

Die DNA-Amplifikation wird erfindungsgemäß in einem *Random Primed* Sequenz-Amplifikationverfahren (RPSA) durchgeführt, d.h. das Priming der Amplifikationsreaktionen erfolgt zufällig, z.B. über Primer mit zufällig ausgewählter Sequenz (*Random Primer*). Unter einer Random-Primed Sequenz-Amplifikationsverfahren wird vor dem Hintergrund der vorliegenden Erfindung die Amplifikation der genomischen DNA verstanden, wobei die verwendeten Primer in zufälliger Weise an die - vorzugsweise genomische - DNA binden. Die Zufälligkeit der Primerbindung an die - vorzugsweise genomische - DNA kann durch verschiedene Möglichkeiten hergestellt werden: Es können *Random Primer* zur Amplifikation eingesetzt werden. *Random Primer* haben z.B. für einen Hexamer-Primer die Sequenz NNNNNN, wobei N für ein beliebiges Nukleotid steht. Dadurch können *Random Primer* alle möglichen Sequenzen enthalten.
Alternativ können auch Primer mit degenerierte Sequenzen eingesetzt werden. Diese Primer können z.B. bestimmte Sequenzmotive beinhalten, wobei an einigen Positionen des Primers zufällige Sequenzen eingestreut sind.
Es können auch Primer mit einer bestimmten Sequenz eingesetzt werden, wobei sichergestellt werden muss, dass diese Primer häufig genug an die Ziel-DNA binden. Dies kann z.B. dadurch sichergestellt werden, dass diese Primer entweder kurz sind oder die Primerbindungsbedingungen so eingestellt werden, dass eine unspezifische Bindung erlaubt wird.
Bei einer RPSA wird ein Großteil einer genomischen Nukleinsäure amplifiziert. Wenn mehrere verschiedene genomischen Nukleinsäuren als Template-Nukleinsäure vorliegen, können die Reaktionsbedingungen so gewählt sein, dass alle genomischen Nukleinsäuren, nur eine genomische Nukleinsäure, aber mindestens ein komplexer Teil der genomischen Nukleinsäure der Template-Nukleinsäure amplifiziert wird. Die Komplexität des amplifizierten Teils der genomischen Nukleinsäure ist zwischen 10.000 und 100.000 nt, besonders bevorzugt 100.000 bis 1.000.000, in einer anderen besonders bevorzugten Ausführungsform größer 1.000.000 nt. Beispiele für RPSA-Verfahren sind *Multiple-Displacement-*Amplifikation (MDA), *Rolling-Circle-Amplification* (RCA), *Random-primed* PCR Methoden wie *Degenerate Oligonucleotide Primer -* PCR (DOP-PCR) und *Primer extension preamplification* PCR (PEP-PCR). Andere geeignete PCR-Verfahren fügen zum Beispiel an DNA-Fragmente, die z.B. durch Schneiden der DNA mit Restriktionsendonukleasen oder durch Ultraschallbehandlung entstanden sind, Primerbindungsstellen an. Weitere geeignete PCR-Verfahren verwenden in einem ersten Schritt Primer, die durch ihr 3'-Ende eine zufällige Primerbindung verursachen mit ihrem 5'-Ende jedoch eine spezifische Primerbindungstelle einführen. Im Anschluss findet eine PCR mit den Primern statt, die an spezifischen Primerbindungsstelle hybridisieren. Auch dieses Prinzip kann in einer erfindungsgemäßen RPSA durchgeführt werden.

In einer besonderen Ausführungsform der Erfindung erfolgt also nach Behandlung der DNA in einer Probe eine RPSA zur Amplifikation der an den Methylierungsstellen geschnittenen DNA. Vorzugsweise wird in dieser Ausführungsform keine Ligationsreaktion nach der Restriktion mit der methylierungsabhängigen Nuklease und vor der RPSA durchgeführt.

Polymerasen sind Enzyme, welche die Bildung von Phosphodiester-Bindungen zwischen einzelnen Nukleotiden innerhalb eines Nukleinsäurestrangs katalysieren (z.B. DNA- und RNA-Polymerasen). Neben hitzelabilen Polymerasen können auch nicht-hitzelabile Polymerasen verwendet werden. Besonders bevorzugt sind alle hitzelabilen und nicht-hitzelabilen Polymerasen, die bei den gewählten Versuchsbedingungen eine *Strand-Displacement-*Aktivität zeigen. Entsprechende Polymerasen sind kommerziell erhältlich und dem Fachmann bekannt.

Unter einer Amplifikation einer Nukleinsäure wird die Vermehrung des Templates um mindestens den Faktor 2 oder mehr verstanden. Hierzu kann die Nukleinsäure linear oder exponentiell vermehrt werden. Eine lineare Amplifikation erreicht man beispielsweise mittels RCA in Gegenwart von Primern, die auf dem Target Circle nur mit einer spezifischen Sequenz hybridisieren. Eine exponentielle Amplifikation erreicht man beispielsweise über RCA mit Primern, wobei die Primer mit mindestens 2 Bindestellen auf dem Target Circle hybridisieren oder aber mit mindestens einer Bindestelle auf dem Target Circle und mindestens einer Bindestelle auf dem komplementären Strang hybridisieren. Weitere lineare und exponentielle für die vorliegende Erfindung geeignete Amplifikationsverfahren sind dem Fachmann geläufig, wie z.B. MDA oder PCR.

Unter einer isothermalen Reaktion wird erfindungsgemäß eine Reaktion verstanden, die bei nur einer Temperatur durchgeführt wird. Wird die Reaktion vor Beginn (z.B. auf Eis) oder nach Ablauf der Reaktion (z.B. zur Inaktivierung von Reaktionskomponenten oder Enzymen) auf eine andere Temperatur gebracht, wird die Reaktion immer noch isothermal genannt, solange die eigentliche Reaktion bei einer konstanten Temperatur durchgeführt wird. Eine Temperatur wird auch dann als konstant verstanden, wenn die Schwankung der Temperatur +/- 10°C, vorzugsweise +/- 5°C, nicht überschreitet.

Unter Primer im Sinne der vorliegenden Erfindung wird ein Molekül verstanden, das als Startstelle für ein Enzym mit Nukleinsäure-Polymerase-Aktivität dient. Dieser Primer kann ein Protein, eine Nukleinsäure oder ein anderes Molekül sein, das sich dem Fachmann als Polymerase-Startstelle geeignet erweist. Dieses Molekül kann durch intermolekulare aber auch durch intramolekulare Wechselwirkung als Startstelle dienen. Im Falle von Nukleinsäure-Primern müssen diese nicht, können aber über ihre gesamte Länge mit der Template-Nukleinsäure hybridisieren. Bevorzugt sind Nukleinsäure-Primer, insbesondere Oligonucleotide.

Besonders bevorzugt ist die Verwendung von *Random-Primern* für die Amplifikation der DNA, d.h. ein Primergemisch umfassend mehrere unterschiedliche Primer zufälliger Sequenz.

Es können neben *Random*-*Primern* auch andere Primer zur Amplifikation der Nukleinsäure(n) zur Anwendung kommen. Wie erwähnt können auch degenerierte und/oder sequenzspezifische Primer zu Amplifikation der DNA verwendet werden.

Die zur Amplifikation verwendeten Primer umfassen typischerweise 4 bis 35 Nukleotide, bevorzugt zwischen 5 bis 25 Nukleotide, besonders bevorzugt 6 bis 15 Nukleotide.

In einer bevorzugten Ausführungsform umfasst das erfindungsgemäße Verfahren den zusätzlichen Schritt

### (iv) Detektion mindestens eines Sequenzabschnittes der amplifizierten DNA.

Die Detektion umfasst dabei vorzugsweise die Quantifizierung mindestens eines Sequenzabschnitts (Locus) der amplifizierten DNA. Typischerweise werden mehrere verschiedene Loci gleichzeitig in einem Multiplex-Verfahren detektiert und können dadurch quantifiziert werden. D.h. in Schritt (iv) des erfindungsgemäßen Verfahrens erfolgt vorzugsweise die Quantifizierung der in Schritt (iii) amplifizierten Nukleinsäure über mindestens einen bekannten Sequenzbereich. Zur Quantifizierung des mindestens einen bekannten Sequenzbereich können z.B. spezifische Sonde und/oder sequenzspezifische Primer verwendet werden. Zur Quantifizierung können ebenfalls Doppelstrang-spezifische Fluoreszenzfarbstoffe und/oder mindestens eine spezifische Sonde verwendet werden.

Dabei kann die Quantifizierung der DNA mit einem Hybridisierungs-vermittelten oder einem Sequenzierverfahren erfolgen. Zu den, dem Fachmann bekannten Hybridisierungsvermittelten Verfahren, gehören beispielsweise quantitative Polymerase-Kettenreaktion (PCR), *Real-time* PCR, *Strand Displacement* Amplifikation (SDA), *Transcription Mediated Amplification* (TMA), *Helicase-dependent Amplification* (HDA), *Recombinase Polymerase Amplification* (RPA), *Loop-mediated Isothermal Amplification* (LAMP), *SMart-Amplification Process* (SMAP), oder auch Microarray-basierte Verfahren (z.B. Affymetrix, Illumina, Agilent). Microarray-basierte Verfahren (kurz: Microarray-Verfahren) sind bevorzugte Hybridisierungs-vermittelte Verfahren im Rahmen der vorliegenden Erfindung. Unter Microarray-Verfahren versteht man Verfahren, bei denen 10 oder mehr Nukleinsäuresequenzen parallel an Oberflächen nachgewiesen werden. Diese Oberflächen tragen in der Regel Nukleinsäure Sequenzen, die zum Nachweis von 10 oder mehr Nukleinsäuresequenzen verwendet werden. Die an den Oberflächen immobilisierten Sequenzen müssen nicht notwendigerweise Nukleinsäuren sein, sondern können zum Beispiel auch modifizierte Nukleinsäuren oder auch PNAs sein, aber auch andere Moleküle kommen in Frage. Als Oberflächen kommen in Microarray Verfahren insbesondere gekrümmte oder planare Oberflächen verschiedener Materialien zum Einsatz. Zu den DNA-Sequenzierverfahren gehören beispielsweise *Pyrosequencing* (Biotage AB, 454 Life Sciences (Roche), Solexa® (Illumina® Inc.) oder *SOLiD Sequencing* (Applied Biosystems). Dem Fachmann sind weitere geeignete Quantifizierungsverfahren bekannt.

Besonders bevorzugt ist es, dass die Detektion von einem oder mehreren Sequenzabschnitten der amplifizierten DNA mittels einer quantitativen *real-time* PCR durchgeführt wird. Weiterhin ist es besonders bevorzugt, dass die Detektion von einem oder mehreren Sequenzabschnitten der amplifizierten DNA mittels eines Microarray-Verfahrens durchgeführt wird.

Besonders bevorzugt ist ebenfalls, dass die Detektion von einem oder mehreren Sequenzabschnitten der amplifizierten DNA mittels eines Sequenzierverfahrens durchgeführt wird.

In einer besonderen Ausführungsform des erfindungsgemäßen Verfahrens wird die Quantität von einem oder mehreren Sequenzabschnitten der DNA in der mit der methylierungsabhängigen Nuklease behandelten Probe mit der Quantität dieses oder dieser Sequenzabschnitte der DNA in einer Kontrollprobe verglichen, die nicht mit einer methylierungsabhängigen Nuklease behandelt wurde.

Die Quantität bestimmter Sequenzabschnitte (Loci) in einer Probe bzw. auf einer DNA kann beispielsweise als Schwellenwert-Zyklus (*Threshold cycle* (C_{T})) ausgedrückt werden, wenn die Quantifizierung mittels *real-time* PCR durchgeführt wird. Der C_{T}-Wert gibt dabei an, in welchem Zyklus der PCR die jeweils für eine bestimmte Sequenz indikativen Fluoreszenzwerte oberhalb des messbaren Schwellenwertes liegen und sind daher ein Maß dafür, wie viel DNA der bestimmten Sequenz ursprünglich in der Probe war: Ein niedriger C_{T}-Wert deutet auf eine größere ursprüngliche Menge der bestimmten DNA-Sequenz in der Probe hin als ein höherer C_{T}-Wert, da weniger Amplifikations-Zyklen nötig waren, um die diese Sequenz in der Probe zu detektieren. Kennt man die Effizienz eines bestimmten Amplifikationssystems, so kann man durch Vergleich mit Standardwerten aus einem C_{T}-Wert auf die ursprünglich vorhandene Menge der bestimmten DNA-Sequenz in der Probe rückrechnen. Die C_{T}-Werte nach Behandlung der Probe mit der methylierungsabhängigen Nuklease können beispielsweise mit entsprechenden Werten ohne Behandlung der Probe mit der methylierungsabhängigen Nuklease verglichen werden. Dies kann etwa durch das Bilden der Differenz ("Delta-C_{T}") von C_{T}^{unbehandelt} - C_{T}^{behandelt} geschehen. Je kleiner diese Differenz ist, desto größer ist die Distanz der entsprechenden DNA-Sequenz zu einer Methylierungsstelle. Anders ausgedrückt: Je näher ein Sequenzabschnitt an einer Methylierungsstelle in der DNA liegt, die in dem erfindungsgemäßen Verfahren von der methylierungsabhängigen Nuklease geschnitten wird, desto weniger effizient wird dieser Sequenzabschnitt amplifiziert werden. Im Extremfall ist eine Amplifikation des betroffenen Sequenzabschnitts überhaupt nicht möglich, insbesondere, wenn dieser Sequenzabschnitt selbst methyliert war und daher von der methylierungsabhängigen Nuklease geschnitten wird.

Um die Vergleichbarkeit der Mengen bestimmter DNA-Sequenzabschnitte aus mit der methylierungsabhängigen Nuklease behandelten und unbehandelten Proben zu gewährleisten, sollten die Bedingungen, unter denen Amplifikation und Quantifizierung bzw. Detektion stattfinden, jeweils möglichst identisch sein. D.h. es ist vorteilhaft, das erfindungsgemäße Verfahren parallel auch ohne den Zusatz der methylierungsabhängigen Nuklease zur Probe durchzuführen, also ohne den Schritt (ii).

Wie erwähnt entstehen durch das erfindungsgemäße Verfahren mehr Amplifikate aus den zentralen Bereichen eines mittels der methylierungsabhängigen Nuklease geschnittenen DNA-Fragmentes als aus den randständigen Bereichen. Die genaue Position der methylierten Stelle(n), d.h. der Schnittstellen, muss nicht unbedingt bekannt sein. Wählt man nun einen beliebigen Sequenzbereich zur Analyse aus, so kann man selbst dann eine Aussage über die Methylierung machen, wenn der analysierte Sequenzbereich nur in der Nähe der methylierten Stelle liegt. Eine solche Analyse kann keine Angabe darüber machen, wie stark eine bestimmte Stelle methyliert ist. Es kann mit einer solchen Analyse also keine Aussage darüber getroffen werden, ob eine Sequenz im Genom zu z.B. einem gewissen Anteil methyliert ist, sondern nur angegeben werden, ob es in der Umgebung der analysierten Sequenz generell methylierte Regionen gibt. Daher dient diese Analyse vornehmlich der Feststellung des globalen Methylierungsmusters und nicht der Quantifizierung des Methylierungsgrades definierter Sequenzen. In besonderen Ausführungsformen des erfindungsgemäßen Verfahren kann sich somit an die Behandlung der DNA mit der methylierungsabhängigen Nuklease und anschließender Amplifikation eine Analyse der Sequenzrepräsentation durchgeführt werden.

Die DNA-Polymerase, die während einer PCR oder einer quantitativen (*Real-Time*) PCR (qRT-PCT) im Rahmen des erfindungsgemäßen Verfahren verwendet wird, ist vorzugsweise eine Polymerase aus einem thermophilen Organismus oder ist eine thermostabile Polymerase oder ist eine Polymerase ausgewählt aus der Gruppe von *Thermus thermo*-*philus* (Tth) DNA Polymerase, *Thermus acquaticus* (Taq) DNA Polymerase, *Thermotoga maritima* (Tma) DNA Polymerase, *Thermococcus litoralis* (Tli) DNA Polymerase, *Pyrococcus furiosus* (Pfu) DNA Polymerase, *Pyrococcus woesei* (Pwo) DNA Polymerase, *Pyrococcus kodakaraensis* KOD DNA Polymerase, *Thermus filiformis* (Tfi) DNA Polymerase, *Sulfolobus solfataricus* Dpo4 DNA Polymerase, *Thermus pacificus* (Tpac) DNA Polymerase, *Thermus eggertsonii* (Teg) DNA Polymerase, *Thermus brockianus* (Tbr) und *Thermus flavus* (Tfl) DNA Polymerase.

In der qRT-PCR können fluoreszenzmarkierte Primer und/oder Sonden verwendet werden, z.B. LightCycler-Sonden (Roche), TaqMan Sonden (Roche), Molecular beacons, Scorpion-Primer, Sunrise-Primer, LUX-Primer oder Amplifluor-Primer. Sonden und/oder Primer können z.B. kovalent oder nicht-kovalent gebundene Fluoreszenzfarbstoffe beispielsweise Fluoresceinisothiocyanate (FITC), 6-Carboxyfluorescein (FAM), Xanthen, Rhodamine, ,6-Carboxy-2',4',7',4,7-hexachlorofluorescein (HEX), 6-Carboxy-4',5'-dichloro-2',7'-dimethodyfluorescein (JOE), N,N,N',N'-Tetramethyl-6-carboxyrhodamine (TAMRA), 6-Carboxy-X-rhodamine (ROX), 5-Carboxyrhodamine-6G (R6G5), 6-carboxyrhodamine-6G (RG6), Rhodamine 110; Coumarine, wie Umbelliferone, Benzimide, wie Hoechst 33258; Phenanthridine, wie Texas Red, Ethidiumbromide, Acridinfarbstoffe, Carbazolfarbstoffe, Phenoxazinefarbstoffe, Porphyrinfarbstoffe, Polymethinfarbstoffe, Cyaninfarbstoffe, wie Cy3, Cy5, Cy7, SYBR-Green, BODIPY-Farbstoffe, Quinolinfarbstoffe und Alexa Farbstoffe enthalten.

Dem Fachmann ist bekannt, dass in qRT-PCR auch unabhängig von Primern und Sonden, Doppelstrang-spezifische Fluoreszenzfarbstoffe, z.B. Ethidiumbromid, SYBR Green, PicoGreen, RiboGreen etc. verwendet werden können.

Dem Fachmann sind die geeigneten Bedingungen für eine quantitative PCR. Dies betrifft z.B. das Primer-Design, die Wahl von geeigneten Prozessierungstemperaturen (Denaturierung, Primer Annealing, Elongation), die Zahl der PCR-Zyklen, die Pufferbedingungen.

Die DNA ist im Kontext der vorliegenden Erfindung insbesondere genomische DNA. Unter genomischer DNA wird eine Deoxyribonukleinsäure verstanden, die aus Organismen gewonnen werden kann und teilweise methyliert vorliegt. Die Methylierung kann unterschiedliche Basen und unterschiedliche Positionen betreffen. Die genomische DNA kann z.B. durch Lyse und/oder Reinigung aus Organismen gewonnen worden sein.

Die zu analysierende Nukleinsäure kann unterschiedlichen Ursprungs sein. Sie kann z.B. aus einem oder mehreren Organismen ausgewählt aus der Gruppe umfassend Viren, Phagen, Bakterien, Eukaryoten, Pflanzen, Pilze und Tiere (z.B. Säugetiere, insbesondere Primaten) isoliert worden sein. Die Nukleinsäure kann z.B. auch aus Zellorganellen stammen. Weiter kann die zu analysierende Nukleinsäure Bestandteil von Proben sein. Solche Proben können ebenfalls unterschiedlicher Herkunft sein. So betrifft das erfindungsgemäße Verfahren auch die Analyse von Nukleinsäuren, die in Proben aus Körperflüssigkeiten, Umweltproben oder Nahrungsmittelproben enthalten sind.

Unter Organismen versteht man im Zusammenhang der vorliegenden Erfindung jegliche Form von organischen Hüllen, die Nukleinsäuren enthalten. Dazu gehören z.B. Viren, Phagen, prokaryotische und eukaryotische Zellen, Zellverbände oder ganze Organismen. Diese Organismen können lebend oder tot eingesetzt werden. Diese Organismen können in Lösung, pelletiert oder auch mit festen Phasen assoziiert oder gebunden sein. Unter "Organismen" können auch mehrere Organismen derselben Art, mehrere Organismen unterschiedler Arten oder auch ein Organismus in Einzahl gemeint sein.

Wie erwähnt, kann im erfindungsgemäßen Verfahren auch eine Lyse des die Nukleinsäure enthaltenden Organismus, Zelle oder Gewebe vor der Amplifikation nötig sein. Unter dem Begriff Lyse wird im Rahmen der vorliegenden Erfindung ein Prozess verstanden, der dazu führt, dass Nukleinsäuren und/oder Proteine aus einem Probenmaterial heraus in die Umgebung abgegeben werden. Dabei kann die Struktur des Probenmaterials zerstört werden, z.B. die Hülle des Probenmaterials aufgelöst werden. Unter dem Begriff "Lyse" wird im Rahmen der vorliegenden Erfindung auch verstanden, dass die Nukleinsäure aus dem Probenmaterial durch kleine Öffnungen, z.B. Poren etc. in der Hülle des Probenmaterials austreten können, ohne dass dabei die Struktur der Probenmaterials zu zerstören. Beispielsweise können durch Lysereagenzien Poren erzeugt werden. Des Weiteren ist im Rahmen der vorliegenden Erfindung unter dem Begriff "Lyse" zu verstehen, dass Nukleinsäuren und/oder Proteine vom Probenmaterial, das schon strukturell zerstört erscheinen oder kleine Öffnungen aufweisen, durch die Verwendung eines Additivs herausgespült werden können. Durch die Lyse wird ein Lysat erzeugt. Das Lysat kann Probenmaterial verschiedener oder eines einzelnen Organismus, verschiedener oder einer einzelner Zelle oder verschiedener oder eines einzelnen Gewebes enthalten.

Unter Reinigung von DNA wird verstanden, dass die DNA von anderen Umgebungsstoffen getrennt wird. Das bedeutet, dass die Probe nach Reinigung der DNA weniger komplex in Bezug auf ihre Inhaltsstoffe ist.

Die vorliegende Beschreibung offenbart außerdem ein Kit zur selektiven Anreicherung von nicht-methyliertem Sequenzabschnitten von genomischer DNA, umfassend
- eine DNA-Polymerase,
- einer methylierungsabhängige Nuklease
- optional: einen Puffer für die Amplifikationsreaktion (z.B. enthaltend Puffersubstanz, dNTPs und/oder Primer)
- optional: einen Puffer für die endonucleolytische Spaltung von methylierten Sequenzabschnitten durch die methylierungsabhängige Nuklease.
Weiterhin betrifft die vorliegende Beschreibung auch ein Kit zur Bestimmung des globalen Methylierungsmusters einer genomischen DNA, umfassend
- eine DNA-Polymerase,
- einer methylierungsabhängige Nuklease
- optional: einen Puffer für die Amplifikationsreaktion (z.B. enthaltend Puffersubstanz, dNTPs und/oder Primer)
- optional: einen Puffer für die endonucleolytische Spaltung von methylierten Sequenzabschnitten durch die methylierungsabhängige Nuklease.

Die DNA-Polymerase der hier beschriebenen Kits ist vorzugsweise eine Polymerase aus einem thermophilen Organismus oder ist eine thermostabile Polymerase oder ist eine Polymerase ausgewählt aus der Gruppe von *Thermus thermo*-*philus* (Tth) DNA Polymerase, *Thermus acquaticus* (Taq) DNA Polymerase, *Thermotoga maritima* (Tma) DNA Polymerase, *Thermococcus litoralis* (Tli) DNA Polymerase, *Pyrococcus furiosus* (Pfu) DNA Polymerase, *Pyrococcus woesei* (Pwo) DNA Polymerase, *Pyrococcus kodakaraensis* KOD DNA Polymerase, *Thermus filiformis* (Tfi) DNA Polymerase, *Sulfolobus solfataricus* Dpo4 DNA Polymerase, *Thermus pacificus* (Tpac) DNA Polymerase, *Thermus eggertsonii* (Teg) DNA Polymerase, *Thermus brockianus* (Tbr) und *Thermus flavus* (Tfl) DNA Polymerase.

Die methylierungsabhängige Nuklease der hier beschriebenen Kits ist vorzugsweise ausgewählt aus der Gruppe bestehend aus McrBC, McrA, DpnI, BisI, BlsI, GlaI, GluI, MalI und PcsI. Bevorzugt sind McrBC und McrA, besonders bevorzugt ist McrBC.
Die erfindungsgemäßen Verfahren und Kits können z.B. zur selektiven Präparation, d.h. selektiven Anreicherung, von nicht-methylierten Sequenzabschnitten von genomischer DNA. Außerdem können sie zur Analyse des globalen Methylierungsmusters in genomischer DNA verwendet werden.

### Beschreibung der Zeichnungen

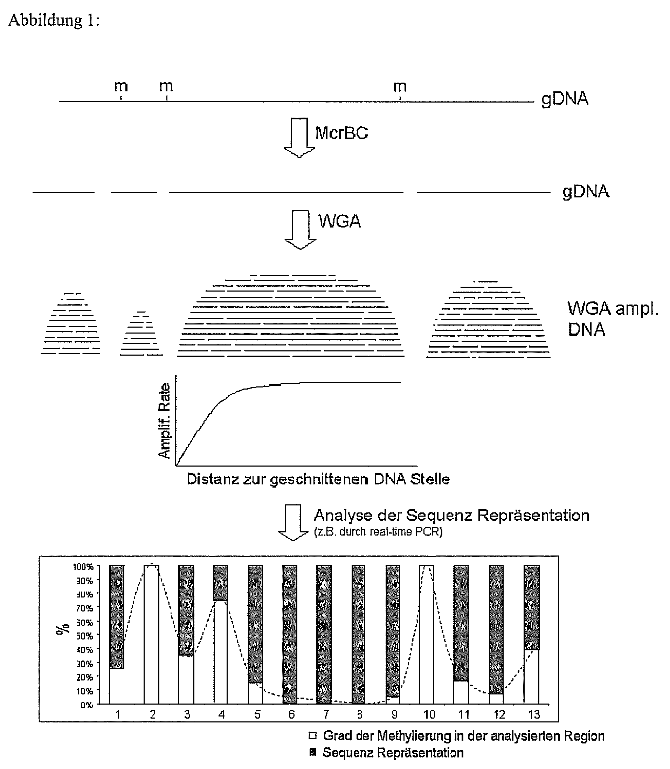

Abbildung 1: Illustration einer beispielhaften Ausführungsform des erfindungsgemäßen Verfahrens: Gezeigt ist eine genomische DNA ("gDNA") bestehend aus nicht-methylierten und methylierten Genomabschnitten. Die methylierten Stellen sind mit "m" gekennzeichnet. In einem ersten Schritt erfolgt eine nukleolytische Spaltung der gDNA nach Erkennung der methylierten Sequenzabschnitte durch die methylierungsabhängige Nuklease McrBC. In einem zweiten Schritt erfolgt die Amplifikation der geschnitten DNA. In dieser beispielhaften Ausführung wird eine Whole-Genome-Amplification (WGA) vorgenommen. Die Schar von amplifizierten DNA Molekülen ist angegeben ("WGA ampl. DNA"). Aus den zentralen Bereichen entstehen deutlich mehr Amplifikate geschnittene DNA Fragmentes als aus den randständigen Bereichen.

### Beispiele

### Beispiel 1: Beispielhafte Ausführung des Verfahren

Eine genomische DNA (als "gDNA" gekennzeichnet) besteht aus nicht-methylierten und methylierte Genomabschnitten. Die methylierten Stellen sind in Abbildung 1 mit "m" gekennzeichnet. In einem ersten Schritt erfolgt eine nukleolytische Spaltung der gDNA nach Erkennung der methylierten Sequenzabschnitte durch eine methylierungsabhängige Nuklease (in der Abbildung mit "McrBC" gekennzeichnet). In einem zweiten Schritt erfolgt die Amplifikation der geschnittenen DNA. In dieser beispielhaften Ausführung wird eine Whole-Genome-Amplification vorgenommen (in der Abbildung mit "WGA" gekennzeichnet). Die Schar von amplifizierten DNA Molekülen ist angegeben ("WGA ampl.DNA"). Man kann deutlich erkennen, dass mehr Amplifikate entstehen aus den zentralen Bereichen eines geschnittenen DNA Fragmentes als aus den randständigen Bereichen. Dies führt zu dem Vorteil, dass die genaue Position der methylierten Stelle nicht unbedingt bekannt sein muss. Wählt man nun einen Bereich beliebigen Sequenzbereich zur Analyse aus, so kann man selbst dann eine Aussage über die Methylierung machen, wenn der analysierte Sequenzbereich nur in der Nähe der methylierten Stelle liegt. Eine solche Analyse kann keine Angabe darüber machen, wie stark eine bestimmte Stelle methyliert ist (es kann also keine Aussage darüber getroffen werden, ob eine Sequenz im Genom zu z.B. 35% methyliert ist), sondern gibt lediglich an, ob es in der Umgebung der analysierten Sequenz generell methylierte Regionen gibt. Daher dient diese Analyse vorzugsweise der Feststellung des globalen Methylierungsmusters.

### Beispiel 2: Beispiel zur Bestimmung der Methylierung in genomischen Regionen

**Versuchsbeschreibung:** Genomische DNA wurde mittels des QIAamp Kits (QIAGEN) aus HepG2 Zellen isoliert. 1 µg der DNA wurde in einen Ansatz mit McrBC Enzym überführt: Dieser Ansatz ("+McrBC Ansatz") enthielt 1 µg DNA, 0,5 U/µl McrBC(NEB), 1xNEB2 Buffer (NEB), 100 ng/µl BSA und 1 mM GTP. Ein weiterer Ansatz ("-McrBC Ansatz") enthielt die gleichen Komponenten, jedoch kein McrBC Enzym. Beide Ansätze wurden für 2 h bei 37°C inkubiert gefolgt von einer Inaktivierung bei 65°C für 20 min. Anschließend wurden den Ansätzen 10 ng für eine WGA Reaktionen entnommen. Die WGA Reaktion wurde mit REPLI-g Midi Reagenzien nach dem REPLI-g Midi Protokoll für gereinigte DNA vorgenommen. Die WGA erfolgte für 8 h bei 30°C gefolgt von einer Inaktivierung von 5 min bei 65°C. Um die Sequenzrepräsentanz nach erfolgter WGA zu messen wurde eine Real-time PCR Analyse durchgeführt. Hier wurden drei verschiedene genomische Loci analysiert. Die Primer für die Analyse sind in der Tabelle 1 angegeben.

**Tabelle 1: Verwendete Primer für die Loci a, b und c aus Beispiel 2**

| | Primer |
|---|---|
| Locus a | TTC CCA CTC AAA ACT CCC AC |
| | ACA GGA ATG AGG GCA GCT AA |
| Locus b | TGCCCGCGTCCGTCCGTGAAA |
| | AGTCTCCGTCGCCGTCCTCGTC |
| Locus c | GGT AGG ATG ATT CTA GAA TGA CA |
| | GCC CAA ATT GGC TTC TTT TT |

Für die *Real-time* PCR wurden QuantiTect Sybr Green Reagenzien (QIAGEN) und 10 ng der jeweiligen WGA DNA in einer 20 µl PCR Reaktion verwendet. Die Primerkonzentration in den Analysen lag bei 0,4 µM. Die Threshold-Cycle (C_{T}-Werte) wurden aufgenommen und sind in der untenstehenden Tabelle 2 wiedergegeben.

**Tabelle 2: Bestimmte Threshold-Werte für (C_{T}-Werte) für die Loci a, b und c aus Beispiel 2**

| | | Locus a | Locus b | Locus c |
|---|---|---|---|---|
| - McrBC | WGA 1 | 23,53 | 20,38 | 25,13 |
| | WGA 2 | 23,38 | 20,30 | 25,25 |
| + McrBC | WGA 3 | 31,35 | 28,04 | 25,22 |
| | WGA 4 | 30,66 | 27,61 | 24,13 |

Zieht man den Mittelwert aus WGA 3und 4 von dem Mittelwert aus WGA 1 und 2 ab, so erhält man den Sequenzrepräsentanzunterschied Delta-C_{T} (Tabelle 3).

**Tabelle 3: Delta-C_{T}-Werte für die Loci a, b und c aus Beispiel 2**

| | Locus a | Locus b | Locus c |
|---|---|---|---|
| Delta-C_{T} | 7,55 | 7,48 | -0,52 |

**Ergebnis:** In Tabelle 2 kann man erkennen, dass die C_{T}-Werte (Locus a und Locus b) für die WGA Reaktionen 3 und 4 ("+ McrBC Ansätze") höher sind als für die WGA Reaktionen 1 und 2 ("- McrBC Ansätze"). Die höheren C_{T}-Werte (deutlich auch durch die Delta-C_{T} Werte) zeigen eine geringere Sequenzrepräsentanz an. Das bedeutet, dass Locus a und Locus b in einer geringeren Konzentration in der WGA 3 und 4 enthalten sind als in der WGA 1 und 2. Eine geringere Konzentration von Locus a und Locus b in WGA 3 und WGA 4 zeigt, dass das McrBC-Enzym in der Nähe dieser Loci die DNA hydrolytisch geschnitten hat. Da McrBC nur dann schneidet, wenn die Erkennungsstellen des Enzyms in der DNA methyliert sind, kann gefolgert werden, dass die DNA in der Nähe der Loci a und b methyliert war.
Anders ist die Situation bei Locus c: Die C_{T}-Werte sind in den WGA Reaktionen 1-4 vergleichbar. Daraus kann gefolgert werden, dass in der Nähe des Locus c keine methylierten Sequenzen zu finden sind.

### Beispiel 3: Beispiel zur Bestimmung der Methylierung in genomischen Regionen bei verschiedenen genomischen DNAs

**Versuchsbeschreibung:** Genomische DNA wurde mittels des QIAamp Kits (QIAGEN) aus HepG2 Zellen und aus dem Blut vier verschiedener Probanden (B1 bis B4) isoliert. 1 µg der DNA wurde in einen Ansatz mit McrBC Enzym überführt: Dieser Ansatz ("+McrBC Ansatz") enthielt 1 µg DNA, 0,5 U/µl McrBC (NEB), 1xNEB2 Buffer (NEB), 100 ng/µl BSA und 1 mM GTP. Ein weiterer Ansatz ("-McrBC Ansatz") enthielt die gleichen Komponenten, jedoch kein McrBC Enzym. Beide Ansätze wurden für 2 h bei 37°C inkubiert gefolgt von einer Inaktivierung bei 65°C für 20 min. Anschließend wurden den Ansätzen 10 ng für eine WGA Reaktionen entnommen. Die WGA Reaktion wurde mit REPLI-g Midi Reagenzien nach dem REPLI-g Midi Protokoll für gereinigte DNA vorgenommen. Die WGA erfolgte für 8 h bei 30°C gefolgt von einer Inaktivierung von 5 min bei 65°C. Um die Sequenzrepräsentanz nach erfolgter WGA zu messen wurde eine Real-time PCR Analyse durchgeführt. Hier wurden drei verschiedene genomische Loci analysiert. Die Primer für die Analyse sind in Tabelle 4 angegeben.

**Tabelle 4: Verwendete Primer für die Loci a bis g aus Beispiel 3**

| | Primer (5'-3'-Sequenz) | Sequenz-ID |
|---|---|---|
| Locus a | TTCCCACTCAAAACTCCCAC | SEQ ID NO:1 |
| | ACAGGAATGAGGGCAGCTAA | SEQ ID NO:2 |
| Locus b | TGCCCGCGTCCGTCCGTGA.AA | SEQ ID NO:3 |
| | AGTCTCCGTCGCCGTCCTCGTC | SEQ ID NO:4 |
| Locus c | GGTAGGATGATTCTAGAATGACA | SEQ ID NO:5 |
| | GCCCAAATTGGCTTCTTTTT | SEQ ID NO:6 |
| Locus d | GTCTTTAGCTGCTGAGGAAATG | SEQ ID NO:7 |
| | AGCAGAATTCTGCACATGACG | SEQ ID NO:8 |
| Locus e | CAACTGGCCCTGTCGTTCC | SEQ ID NO:9 |
| | CCATGTTGCTGACCCGGTAG | SEQ ID NO:10 |
| Locus f | ACTGGTTGGAGTTGTGGAGACG | SEQ ID NO:11 |
| | TGGAATGCTTGAAGGCTGCTC | SEQ ID NO:12 |
| Locus g | AACTGAATGGCAGTGAAAACA | SEQ ID NO:13 |
| | CCCTAGCCTGTCATTGCTG | SEQ ID NO:14 |

Für die Real-time PCR wurden QuantiTect Sybr Green Reagenzien (QIAGEN) und 10 ng der jeweiligen WGA DNA in einer 20 µl PCR Reaktion verwendet. Die Primerkonzentration in den Analysen lag bei 0,4 µM. Die Threshold-Cycle (C_{T}-Werte) wurden aufgenommen und sind in der untenstehenden Tabelle 5 wiedergegeben.
Zieht man die C_{T}-Werte, die aus den WGA Reaktionen mit vorheriger McrBC Behandlung der genomischen DNA gewonnen wurden, von den C_{T}-Werten ab, die aus den WGA Reaktionen ohne vorheriger McrBC Behandlung der DNA gewonnen wurden, erhält man den Delta-C_{T} Wert. Der Delta-C_{T} Wert ist ein Maß dafür wie stark sich die Repräsentanz der untersuchten Loci zwischen den +McrBC Ansätzen und den -McrBC Ansätzen unterscheidet. Ein sehr hoher Delta-C_{T} Wert zeigt an, dass die Repräsentanz in den +McrBC Ansätzen gegenüber den -McrBC Ansätzen deutlich abgenommen hat.

**Tabelle 5: Delta-C_{T}-Werte für die Loci a bis g aus Beispiel 3**

| | Locus a | Locus b | Locus d | Locus e | Locus f | Locus g |
|---|---|---|---|---|---|---|
| HepG2 | 6,6 | 7,8 | 4,6 | 6,2 | 7,7 | 18,0 |
| B1 | 4,4 | 2,5 | 2,6 | 7,7 | 13,0 | 14,4 |
| B 2 | 3,6 | 3,4 | 2,8 | 7,9 | 10,8 | 10,2 |
| B 3 | 4,0 | 3,3 | 3,4 | 4,4 | 11,1 | 10,0 |
| B 4 | 5,3 | 2,7 | 3,9 | 9,7 | 9,4 | 13,5 |

Ergebnis: Aus der Tabelle der Delta-C_{T} Werte kann man erkennen, dass die Delta-C_{T}-Wert sich ähneln, wenn B1 bis B4 verglichen werden. So liegen die Delta-C_{T} Werte bei Locus b zwischen 2,5 und 3,5. Dagegen ist bei der genomischen DNA von HepG2 Zellen der Delta-C_{T} Wert von Locus b deutlich verschieden von den Delta-C_{T} Werten des Blutes der Spender B1 bis B4. Dies deutet auf ein anderes Methylierungsmuster bei HepG2 Zellen gegenüber den Blut der Probanden B1 bis B4 hin. Bei Locus f findet sich bei HepG2 Zellen ein niedrigerer Delta-C_{T} Wert als im Blut, was auf eine stärkere Methylierung am Ort oder in der Nähe des Locus f im Blut hinweißt.

## Patentansprüche

1. Verfahren zur selektiven Amplifikation von nicht-methylierten Sequenzen einer DNA umfassend die Schritte
(i) Bereitstellen einer Probe umfassend eine DNA, die an mindestens einer Stelle methyliert ist,
(ii) Behandlung der DNA in der Probe mit einer methylierungsabhängigen Nuklease, und
(iii) Isothermale *Random Primed* Sequenz-Amplifikation der mit der methylierungsabhängigen Nuklease geschnittenen DNA;
wobei die Schritte (ii) und (iii) simultan durchgeführt werden.

2. Verfahren nach Anspruch 1, wobei die Amplifikation mittels Strand Displacement Amplifikation durchgeführt wird.

3. Verfahren nach einer der Ansprüche 1 bis 2, umfassend den zusätzlichen Schritt (iv) Detektion mindestens eines Sequenzabschnittes der amplifizierten DNA.

4. Verfahren nach Anspruch 3, wobei die Detektion die Quantifizierung mindestens eines Sequenzabschnitts der amplifizierten DNA umfasst.

5. Verfahren nach Anspruch 4, wobei die Detektion von einem oder mehreren Sequenzabschnitten der amplifizierten DNA durch ein Hybridisierungs-vermittelten Verfahren durchgeführt wird.

6. Verfahren nach Anspruch 5, wobei die Detektion von einem oder mehreren Sequenzabschnitten der amplifizierten DNA mittels einer quantitativen real-time PCR durchgeführt wird.

7. Verfahren nach Anspruch 5, wobei die Detektion von einem oder mehreren Sequenzabschnitten der amplifizierten DNA mittels eines Microarray-basierten Verfahrens durchgeführt wird.

8. Verfahren nach Anspruch 4, wobei die Detektion von einem oder mehreren Sequenzabschnitten der amplifizierten DNA mittels eines Sequenzierungsverfahrens durchgeführt wird.

9. Verfahren nach einem der Ansprüche 3 bis 8, wobei die Quantität von einem oder mehreren Sequenzabschnitten der DNA in der mit der methylierungsabhängigen Nuklease behandelten Probe mit der Quantität dieses oder dieser Sequenzabschnitte der DNA in einer Kontrollprobe verglichen wird, die nicht mit einer methylierungsabhängigen Nuklease behandelt wurde.

10. Verfahren nach einem der vorstehenden Ansprüche, wobei die methylierungsabhängige Nuklease ausgewählt ist aus der Gruppe bestehend aus McrBC, McrA, DpnI, BisI, BlsI, GlaI, GluI, MalI und PcsI.

11. Verfahren nach einem der vorstehenden Ansprüche, wobei die DNA genomische DNA ist.

12. Verwendung des Verfahrens nach einem der Ansprüche 1 bis 11 zur selektiven Präparation (selektive Anreicherung) von nicht- methylierten Sequenzabschnitten von genomischer DNA.

13. Verwendung des Verfahrens nach einem der Ansprüche 1 bis 11 zur Analyse des globalen Methylierungsmusters in genomischer DNA.

## Claims

1. Method for the selective amplification of non-methylated sequences of a DNA comprising the steps of
(i) providing a sample comprising a DNA which is methylated on at least one site,
(ii) treating the DNA in the sample with a methylation-dependent nuclease, and
(iii) performing isothermal random-primed sequence amplification of the DNA which was digested with the methylation-dependent nuclease;
wherein the steps (ii) and (iii) are performed simultaneously.

2. The method according to claim 1, wherein the amplification is performed by strand displacement amplification.

3. The method according to any of claims 1 to 2, comprising the additional step of (iv) detecting at least one sequence segment of the amplified DNA.

4. The method according to claim 3, wherein the detection comprises the quantification of at least one sequence segment of the amplified DNA.

5. The method according to claim 4, wherein the detection of one or several sequence segments of the amplified DNA is performed by a hybridisation-based method.

6. The method according to claim 5, wherein the detection of one or several sequence segments of the amplified DNA is performed by a quantitative real-time PCR.

7. The method according to claim 5, wherein the detection of one or several sequence segments of the amplified DNA is performed by a microarray-based method.

8. The method according to claim 4, wherein detecting one or several sequence segments of the amplified DNA is performed by a sequencing method.

9. The method according to any of claims 3 to 8, wherein the quantity of one or several sequence segments of the amplified DNA in the sample treated with the methylation-dependent nuclease is compared to the quantity of this or these sequence segments of the DNA in a control sample which was not treated with a methylation-dependent nuclease.

10. The method according to any of the preceding claims, wherein the methylation-dependent nuclease is selected from the group consisting of McrBC, McrA, DpnI, BisI, BlsI, GlaI, GluI, MalI and PcsI.

11. The method according to any of the preceding claims, wherein the DNA is genomic DNA.

12. Use of the method according to any of claims 1 to 11 for the selective preparation (selective enrichment) of non-methylated sequence segments of genomic DNA.

13. Use of the method according to any of claims 1 to 11 for the analysis of the global methylation pattern in genomic DNA.

## Revendications

1. Procédé pour l'amplification sélective de séquences non-méthylées d'un ADN comprenant les étapes
(i) fournir un échantillon comprenant un ADN méthylé sur au moins une position,
(ii) traiter l'ADN de l'échantillon avec une nucléase sensible à la méthylation, et
(iii) effectuer une amplification isotherme à amorces aléatoires de séquence de l'ADN digéré avec la nucléase sensible à la méthylation ;
où les étapes (ii) et (iii) sont effectuées simultanément.

2. Le procédé selon la revendication 1, dans lequel l'amplification est effectuée par une amplification par déplacement de brin.

3. Le procédé selon l'une quelconque des revendications 1 à 2, comprenant l'étape additionnelle
(iv) détecter au moins une section de séquence de l'ADN amplifié.

4. Le procédé selon la revendication 3, dans lequel la détection comprend la quantification d'au moins une section de séquence de l'ADN amplifié.

5. Le procédé selon la revendication 4, dans lequel la détection d'une ou plusieurs sections de séquence de l'ADN amplifié est effectuée par un procédé médié par une hybridation.

6. Le procédé selon la revendication 5, dans lequel la détection d'une ou plusieurs sections de séquence de l'ADN amplifié est effectué par une PCR en temps réel quantitative.

7. Le procédé selon la revendication 5, dans lequel la détection d'une ou plusieurs sections de séquence de l'ADN amplifié est effectué par un procédé basé sur le micrarray.

8. Le procédé selon la revendication 4, dans lequel la détection d'une ou plusieurs sections de séquence de l'ADN amplifié est effectuée par un procédé de séquençage.

9. Le procédé selon l'une quelconque des revendications 3 à 8, dans lequel la quantité d'une ou plusieurs sections de l'ADN dans l'échantillon traité avec la nucléase sensible à la méthylation est comparée à la quantité de cette ou de ces sections de séquence d'ADN dans un échantillon contrôle qui n'a pas été traité avec une nucléase sensible à la méthylation.

10. Le procédé selon l'une quelconque des revendications précédentes, dans lequel la nucléase sensible à la méthylation est choisie du groupe consistant en McrBC, McrA, DpnI, BisI, BlsI, Glal, Glul, MalI et PcsI.

11. Le procédé selon l'une quelconque des revendications précédentes, dans lequel l'ADN est de l'ADN génomique.

12. Utilisation du procédé selon l'une quelconque des revendications 1 à 11 pour la préparation sélective (l'enrichissement sélectif) de sections de séquence non-méthylées d'ADN génomique.

13. Utilisation du procédé selon l'une quelconque des revendications 1 à 11 pour l'analyse du motif de méthylation global dans de l'ADN génomique.
